# EUROPEAN PATENT APPLICATION

(11) **EP 1 801 094 A1**
(43) Date of publication of application: **27.06.2007**
(21) Application number: 05793121.4
(22) Date of filing: 11.10.2005
(51) Int. Cl.: C07C 67/32, C07C 69/65, C12P 41/00, C07C 67/343

(54) **METHOD FOR PRODUCING (4E)-5-CHLORO-2-ISOPROPYL-4-PENTENOATE AND OPTICALLY ACTIVE SUBSTANCE THEREOF**

(30) Priority: 15.10.2004 JP 2004301870
(71) Applicant: Asahi Glass Company, Limited, Chiyoda-ku, Tokyo 100-8405 (JP)
(72) Inventor: SAKAEDA, Toshitsugu, Asahi Glass Company, Limited, Ichihara-shi Chiba 290-8566 (JP); MORI, Nobuaki, Asahi Glass Company, Limited, Tokyo 100-8405 (JP); KANOH, Hideto, Seimi Chemical Co., Ltd., Chigasaki-shi Kanagawa 253-8585 (JP)
(74) Representative: Müller-Boré & Partner Patentanwälte
(86) International application number: PCT/JP2005/018704
(87) International publication number: WO 2006/041062

(57) **Abstract**

To provide a process for producing a (4E)-5-chloro-2-isopropyl-4-pentenoate in high yield and efficiently.

A compound (2) is reacted with a base in the presence of an aprotic solvent and then with (1E)-1,3-dichloro-1-propene in the same reaction vessel to obtain a compound (3), and then either of -COOR moieties in the compound (3) is replaced with a hydrogen atom in the same reaction vessel to obtain a compound (4): 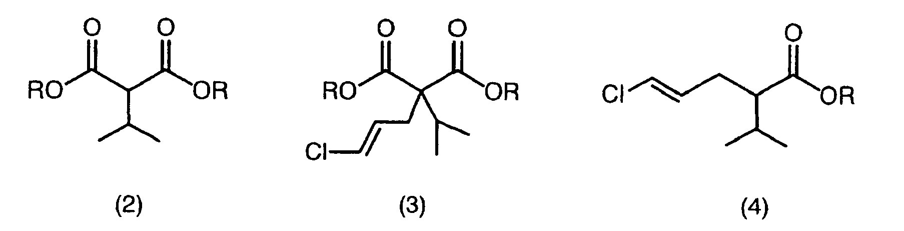 wherein R is a lower alkyl group or an aralkyl group.

## Description

### TECHNICAL FIELD

The present invention relates to processes for producing a (4E)-5-chloro-2-isopropyl-4-pentenoate useful as an intermediate for an agrochemical or a medicine and an optically active form thereof.

### BACKGROUND ART

For an efficient synthesis of a (4E)-5-chloro-2-isopropyl-4-pentenoate useful as an intermediate for an agrochemical or a medicine and its analogues, the following process has been reported by the present applicant.

A process for obtaining a compound of the following formula (4), which comprises reacting a dialkyl malonate with a base in the presence of an aprotic solvent and then with an isopropyl halide to obtain a compound of the following formula (2), and then reacting the compound of the formula (2) with a base in the presence of an aprotic solvent and then with (1E)-1,3-dichloro-1-propene to obtain a compound of the following formula (3), followed by dealkoxycarbonylation of the compound of the formula (3) (see Patent Document 1). 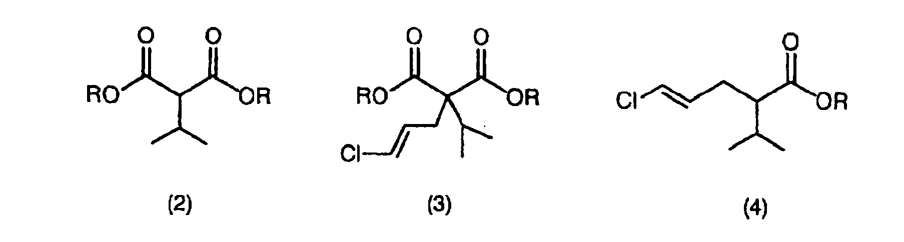 Patent Document 1: WO04/52828

### DISCLOSURE OF THE INVENTION

### PROBLEMS THAT THE INVENTION IS TO SOLVE

The present inventors have conducted studies in order to make the above production process to be a more efficient production process. For example, they considered that addition of water to the reaction solution in order to isolate the compound of the formula (3) or the compound of the formula (4) from the reaction solution containing such compounds, is a cause whereby the solvent and water are mixed to make recovery and reuse of the solvent difficult. Further, they considered that the treatment operation for the isolation tends to cause decrease in the yield, and particularly, addition of water as work-up treatment tends to cause decrease in the yield since the desired compound tends to transfer to the aqueous layer together with the solvent.

It is an object of the present invention to provide processes for producing a (4E)-5-chloro-2-isopropyl-4-pentenoate and its optically active form more efficiently in high yield.

### MEANS OF SOLVING THE PROBLEMS

The present inventors have found that it is possible to solve the above problems by carrying out the reaction to obtain the compound of the formula (3) from the compound of the formula (2) and the dealkoxycarbonylation in the same reaction vessel.

Namely, the present invention provides the following.
1. A process for producing a compound of the following formula (4), which comprises reacting a compound of the following formula (2) with a base (II) in the presence of an aprotic solvent (II), and then with (1E)-1,3-dichloro-1-propene in the same reaction vessel to obtain a compound of the following formula (3), and then carrying out a reaction to replace one of -COOR moieties in the compound of the following formula (3) with a hydrogen atom in the same reaction vessel, wherein R is a lower alkyl group or an aralkyl group.
2. The process according to the above 1, wherein the reaction for conversion from the compound of the formula (3) to the compound of the formula (4), is carried out after the aprotic solvent (II) is distilled off from the reaction product obtained by the reaction with (lE)-1,3-dichloro-1-propene.
3. The process according to the above 2, wherein the reaction for conversion from the compound of the formula (3) to the compound of the formula (4), is carried out in the presence of a salt produced by the preceeding reaction.
4. The process according to any one of the above 1, 2 or 3, wherein the compound of the formula (2) is a compound obtained by reacting a compound of the following formula (1) with a base (I) in the presence of an aprotic solvent (I), and then with an isopropyl halide. R is a lower alkyl group or an aralkyl group.
5. The process according to the above 4, wherein the reaction of the compound of the formula (2) with the base (II), is carried out after the aprotic solvent (I) is distilled off from the reaction product obtained by the reaction with the isopropyl halide.
6. The process according to the above 4 or 5, wherein after the reaction to obtain the compound of the formula (2), the reaction to obtain the compound of the formula (3) is carried out in the same reaction vessel.
7. The process according to the above 4, 5 or 6, wherein the base (I) and the base (II) are the same base.
8. The process according to the above 4, 5, 6 or 7, wherein the aprotic solvent (I) and the aprotic solvent (II) are the same solvent.
9. A process for producing a compound of the following formula (4), which comprises the following steps (a'), (b') and (c'):
   step (a'): a step of reacting a compound of the following formula (1) with an alkali metal alkoxide (I) of the formula M¹OR¹ (wherein M¹ is Na or K, and R¹ is a lower alkyl group) in the presence of an aprotic solvent (I) and then with an isopropyl halide to obtain a reaction product containing a compound of the following formula (2), and then distilling off the aprotic solvent (I) from the reaction product,
   step (b'): a step of reacting the compound of the formula (2) obtained in the step (a') with the alkali metal alkoxide (I) in the presence of an aprotic solvent (I), and then with (1E)-1,3-dichloro-1-propene to obtain a reaction product containing a compound of the following formula (3), and then distilling off the aprotic solvent (I) from the reaction product, and
   step (c'): a step of carrying out a reaction to replace one of -COOR moieties in the compound of the formula (3) obtained in the step (b') with a hydrogen atom, in the presence of an alkali metal chloride of the formula M¹C1 (wherein M¹ is the same as defined above): 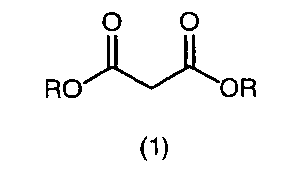 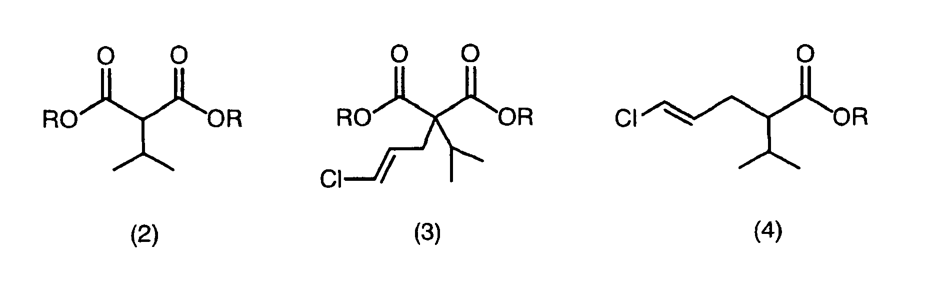
   wherein R is a lower alkyl group or an aralkyl group.
10. The process according to the above 9, wherein the step (c') is a step which comprises heating the compound of the formula (3) obtained in the step (b'), in the presence of an alkali metal chloride of the formula M¹C1 (wherein M¹ is the same as defined above) formed in the step (b') without adding the alkali metal chloride and in the presence of water.
11. A process for producing a compound of the following formula (5a) and/or a compound of the following formula (6a), which comprises optically resolving the compound of the following formula (4) obtained by the process as defined in any one of the above 1 to 10,
   wherein R is the same as defined above.
12. A process for producing a compound of the following formula (5b) and/or a compound of the following formula (6b), which comprises separating, by optical resolution, one of optical isomers of the compound of the following formula (4) obtained by the process as defined in any one of the above 1 to 10, wherein R is the same as defined above.
13. A process for producing a compound of the following formula (5a), which comprises reacting a compound of the formula ROH with the compound of the formula (5b) obtained by the process as defined in the above 12, wherein R is a lower alkyl group or an aralkyl group.
14. The process according to the above 11, 12 or 13,
wherein the optical resolution is carried out by letting an enzyme act on the compound of the following formula (4) :

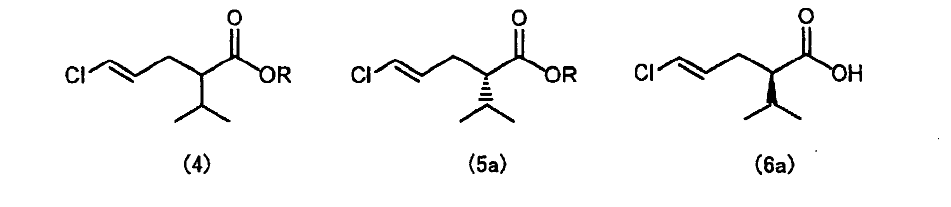

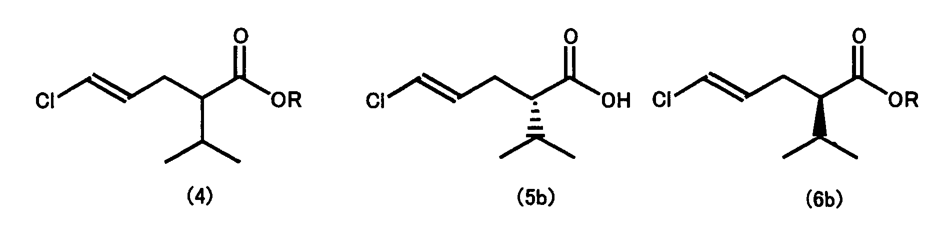

### EFFECT OF THE INVENTION

According to the production process of the present invention, it is possible to efficiently produce a (4E)-5-chloro-2-isopropyl-4-pentenoate useful as an intermediate for an agrochemical or a medicine. Namely, it is possible to recover and reuse the solvent used for quaternization and dealkoxycarbonylation, and thus it is possible to reduce waste water or waste liquid. Further, it is possible to improve the reaction rate or yield over conventional processes.

Further, by optically resolving a (4E)-5-chloro-2-isopropyl-4-pentenoate obtained by the above process, it is possible to obtain a (S)-(4E)-5-chloro-2-isopropyl-4-pentenoate useful as an intermediate for an agrochemical or a medicine in high yield and high enantiomeric excess.

### BEST MODE FOR CARRYING OUT THE INVENTION

Hereinafter, "the compound of the formula (1)" is also referred to as "the compound (1)". Compounds of the other formulae are referred to similarly. Further, unless otherwise specified, pressure is represented by absolute pressure.

The production process described in the present specification is outlined by the following formulae, but is not restricted to the following formulae. In the formulae, R is a lower alkyl group or an aralkyl group (hereinafter the same applies).

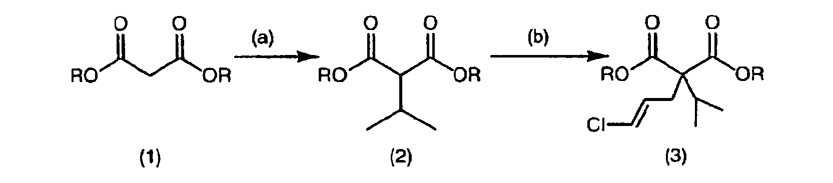

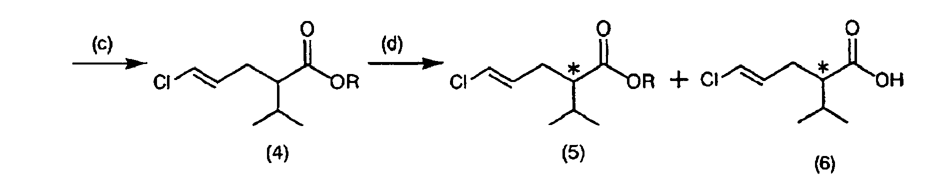

The above production process comprises the following steps (a) to (d):
step (a): a step of reacting a compound (1) with a base (I) in the presence of an aprotic solvent (I) and then with an isopropyl halide to obtain a compound (2),
step (b): a step of reacting the compound (2) with a base (II) in the presence of an aprotic solvent (II) and then with (lE)-1,3-dichloro-1-propene in the same reaction vessel to obtain a compound (3),
step (c): a step of replacing (dealkoxycarbonylating) one of -COOR moieties in the compound (3) with a hydrogen atom in the same reaction vessel as in step (b), to obtain a compound (4), and
step (d): a step for obtaining optically active forms of the compound (4) i.e. a step of optically resolving the compound (4) to obtain a compound (5) and/or a compound (6).

Here, * in the formulae shows that the carbon atom is an asymmetric carbon atom, the absolute configuration of the asymmetric carbon atom in the compound (5) and the absolute configuration of the asymmetric carbon atom in the compound (5) are different each other, and one is R and the other is S (hereinafter the same applies).

In the present invention, R is a lower alkyl group or an aralkyl group.

The lower alkyl group means a C₁₋₄ alkyl group such as a methyl group, an ethyl group, an n-propyl group, an iso-propyl group, an n-butyl group, a sec-butyl group, an iso-butyl group or a tert-butyl group.

The aralkyl group means an aryl-substituted lower alkyl group, preferably a lower alkyl group substituted with one or two aryl groups. The aryl group may, for example, be a phenyl, 1-naphthyl or 2-naphthyl group which may have one or more substituents on the ring. The substituents are preferably lower alkyl groups. The aralkyl group may, for example, be a benzyl group or a diphenylmethyl group.

R is preferably a lower alkyl group, particularly preferably a methyl group.

As the base (I) and the base (II), metal hydrides, metal alkoxides, lithium diisopropylamide (LDA), lithium hexamethyldisilazide, pyridine, triethylamine, inorganic bases and the like may be used, respectively.

It is preferred to use a metal alkoxide of the formula M¹OR¹ (wherein M¹ is Na or K, and R¹ is a lower alkyl group) as the base (I) and a metal alkoxide of the formula M²OR² (wherein M² is Na or K, and R² is a lower alkyl group) as the base (II) because they are highly reactive and handleable and economically advantageous. The bases (I) and (II) are preferably the same.

As the metal alkoxides of the formulae M¹OR¹ and M²OR², sodium methoxide (CH₃ONa), sodium ethoxide (C₂H₅ONa), sodium tert-butoxide (t-C₄H₉ONa) and potassium tert-butoxide (t-C₄H₉OK) may, for example, be mentioned, respectively. Inexpensively available CH₃ONa and C₂H_{S}ONa are preferred.

These metal alkoxides are preferably used in the form of powders or solutions.

When these metal alkoxides are used in the form of solutions, it is preferred that the metal alkoxide of the formula M¹OR¹ is in the form of a solution in an alcohol of the formula R¹OH, and the metal alkoxide of the formula M²OR² is in the form of a solution in an alcohol of the formula R²OH (wherein M¹, M², R¹ and R² are the same as defined above). The concentration of the metal alkoxide in the solution is preferably from 5 to 35 mass%, particularly preferably from 15 to 35 mass%.

When such a metal alkoxide is used in the form of a solution, it may be prepared from an alkali metal and a lower alcohol.

The aprotic solvent (I) and the aprotic solvent (II) may, for example, be aromatic hydrocarbon solvents such as toluene, xylene and benzene; aliphatic hydrocarbon solvents such as hexane and heptane; amide solvents such as dimethylformaide (DMF), dimethylacetamide (DMA), N-methylpyrrolidinone (NMP), and 1,3-dimethyl-2-imidazolidinone (DMI); sulfoxide solvents such as dimethyl sulfoxide (DMSO); sulfone solvents such as sulfolane; or ether solvents such as diethylene glycol dimethyl ether (DME), diglyme, tetrahydrofuran (THF) and t-butyl methyl ether (TBME). These solvents may be used singly or in the form of a solvent mixture of two or more.

The aprotic solvent (I) and the aprotic solvent (II) are preferably made of a solvent consisting of at least one member selected from the group consisting of toluene, xylene, benzene, heptane, DMF, DMA, NMP, DMI, DMSO, DME, THF, TBME and sulfolane. The aprotic solvent (I) and the aprotic solvent (II) are preferably the same solvent.

Further, the aprotic solvent (I) and the aprotic solvent (II) are particularly preferably a solvent mixture of an aromatic hydrocarbon solvent with an amide solvent or sulfolane, and further more preferably a solvent mixture of toluene with sulfolane, in view of prevention of impurity formation, handleability, and recovery and reuse of the solvent. In the solvent mixture, the ratio of the aromatic hydrocarbon solvent to the amide solvent or sulfolane is preferably from 1/2 to 50/1, particularly preferably from 1/2 to 5/1 by aromatic hydrocarbon solvent/(amide solvent or sulfolane) (volume ratio).

Now, the steps (a) to (d) will be described sequentially.

The step (a) is a tertiarization step of reacting a compound (1) with a base (I) in the presence of an aprotic solvent (I), and then with an isopropyl halide to obtain a compound (2).

As the compound (1) (malonic diester), dimethyl malonate, diethyl malonate or diisopropyl malonate may, for example, be used, and dimethyl malonate is preferred.

As the isopropyl halide, isopropyl bromide, isopropyl chloride or isopropyl iodide may, for example, be used, and isopropyl bromide is preferred from the viewpoint of the reactivity and economical efficiency.

Malonic diesters, isopropyl halides and (lE)-1,3-dichloro-1-propene are known-compounds, which are available industrially at low prices. It is usually preferred to use commercial products of these compounds. Such commercial products may usually be used without purification, though they may be purified, if necessary.

In the step (a), the amount of the aprotic solvent (I) is preferably from 0.5 to 20 ml per 1 g of the compound (1).

The amount of the base (I) is preferably 0.9 to 5 times by mol, in particular from 1.0 to 3.0 times by mol, based on the compound (1).

The amount of the isopropyl halide is preferably at least 1.0 time by mol, more preferably from 1.0 to 50.0 times by mol, based on the compound (1) in view of the conversion during the reaction, particularly preferably from 1.0 to 3.0 times by mol in view of handleability, volume efficiency and economical efficiency.

In the step (a), a metal iodide such as sodium iodide (NaI) or potassium iodide (KI), or a metal bromide such as sodium bromide (NaBr) or potassium bromide (KBr) may be added to further increase the reactivity.

When such a metal iodide or bromide is to be added, the amount is preferably from 1 mol% to 100 mol%, particularly preferably from 1 mol% to 10 mol%, based on the isopropyl halide.

In the step (a), the reaction temperature is preferably from +30 to +180°C, particularly preferably from +70°C to +140°C, and the reaction time is preferably from 1 to 30 hours. The reaction pressure is preferably atmospheric pressure or above, particularly preferably atmospheric pressure.

In the step (a), the reaction is preferably carried out by a method (a-1) in which the compound (1), the base (I) and the isopropyl halide are added to the aprotic solvent (I) in this order or by a method (a-2) in which the base (I), the compound (1) and the isopropyl halide are added to the aprotic compound (I) in this order.

In the step (a), as the base (I), a base of the above-mentioned formula M¹OR¹ (M¹ and R¹ are the same as defined above) is preferably used. Also when a base of the formula M¹OR¹ is used, it is preferred to react the compound (1) with the base in the presence of the aprotic solvent (I) first and then with the isopropyl halide in accordance with the method (a-1) or (a-2).

In the reaction using a base of the formula M¹OR¹, an alcohol of the formula R¹OH will be produced as a by-product. For example, when the base is NaOCH₃, methanol will be produced as a by-product, and when the base is NaOC₂H₅, ethanol will be produced as a by-product. The alcohol produced as a by-product is preferably removed from the reaction system before the reaction with the isopropyl halide. Namely, in the step (a), it is preferred to react the compound (1) with the base (I) made of a metal alkoxide of the formula M¹OR¹ in the presence of the aprotic solvent (I) first and then remove the alcohol of the formula R¹OH as a by-product, followed by the reaction with the isopropyl halide.

The alcohol is preferably distilled off, and the method for such distillation is preferably carried out by heating and/or vacuuming the reaction product. Usually, such distillation is preferably carried out by distilling the reaction product under heating before adding the isopropyl halide.

The protic solvent such as the alcohol produced as a by-product is preferably removed from the viewpoint of the conversion and reaction time, though the reaction may proceed even if it remains in the reaction system.

Further, when the isopropyl halide in the step (a) is used in an amount of at least 1 time by mol based on the compound (1), it is preferred that the remaining isopropyl halide is removed from the reaction system after completion of the reaction. If the isopropyl halide remains in a large amount, it is likely to bring about a side reaction such as quaternization of the compound (2) with the isopropyl halide or a reaction of the isopropyl halide with a base of the formula M²OR² (wherein M² and R² are the same as defined above) such as NaOCH₃, in the subsequent step (b). Accordingly, by removing the isopropyl halide from the reaction system, it is possible to prevent such side reaction, whereby it is possible to further enhance the yield of the compound (3) as the desired product of the step (b).

The method for removing the isopropyl halide, is preferably a method of removal by distillation. When the isopropyl halide is to be removed, it is preferred to remove the isopropyl halide until the amount of the isopropyl halide becomes 5 mol% or below, particularly 1 mol% or below, based on the compound (2). Further, the removed isopropyl halide may be reused for the reaction in the step (a).

In the production process of the present invention, the distillation is carried out for removing the solvent from the reaction product containing the compound (2) produced in the step (a). In the present invention, as a work-up treatment step, a work-up treatment step other than the distillation may be carried out, but it is preferred to carry out no work-up treatment except for the distillation so as to achieve the effect of the present invention sufficiently.

As a method for the work-up treatment, the following treatments 1 to 3 may, for example, be mentioned. (Treatment 1) A method which comprises adding water or an aqueous sodium chloride solution and then a water-immiscible organic solvent such as dichloromethane, toluene, ethyl acetate, butyl acetate, t-butyl methyl ether, diisopropyl ether or diethyl ether to the crude reaction solution for extraction, and then concentrating and further distilling the organic layer.
(Treatment 2) A method which comprises washing the organic layer obtained in the treatment 1 with water and/or an aqueous sodium chloride solution and then concentrating and distilling the organic layer. (Treatment 3) A method which comprises cooling the crude reaction solution and distilling it under reduced pressure to isolate the compound (2).

Further, before or after each step in the treatments 1 to 3, filtration or treatment by addition of an adsorbent such as activated carbon may also be carried out.

The step (b) is carried out after the aprotic solvent (I) is distilled off from the product containing the compound (2) produced in the step (a). The step (b) is preferably carried out continuously in the same reaction vessel as the reaction vessel in which the reaction in the step (a) is carried out. Namely, it is preferred that, after the compound (2) is obtained by the step (a), the reaction to obtain the compound (3) in the step (b) is carried out in the same reaction vessel.

It is advantageous for industrial production to carry out the step (a) and the step (b) continuously in the same reaction vessel, whereby the reaction time can be shortened, and the operation is easy. Also in such a case, when the isopropyl halide in the step (a) is used in an amount of at least 1 time by mol based on the compound (1), it is preferred that the isopropyl halide is removed from the reaction system after completion of the step (a) in the same manner as described above.

The step (b) is quarternary alkylation which comprises reacting the compound (2) with a base (II) in the presence of an aprotic solvent (II) and then with (1E)-1,3-dichloro-1-propene to obtain the compound (3).

In the step (b), the aprotic solvent (II) is used in an amount of preferably from 0.5 to 20 ml per 1 g of the compound (2).

The amount of the base (II) is preferably from 0.9 to 5 times by mol, particularly preferably from 0.9 to 3.0 times by mol, further more preferably from 0.9 to 1.5 times by mol, based on the compound (2). When the base (II) is used in excess of 1 time by mol based on the compound (2), the reaction rate advantageously becomes high, but there will be also a high probability that the unreacted base (II) remaining in the reaction system may cause a side reaction with (lE)-1,3-dichloro-1-propene, whereby it is preferred that the amount of the base (II) is determined in consideration of both of the reactivity and the side reaction.

The amount of (1E)-1,3-dichloro-1-propene is preferably from 0.9 to 50.0 times by mol, particularly preferably from 0.1 to 3.0 times by mol, based on the compound (2) from the viewpoint of handleability, volume efficiency and economical efficiency. When (lE)-1,3-dichloro-1-propene is used in excess, it may be recovered for reuse in the step (b).

In the step (b), the reaction is preferably carried out by a method (b-1) in which the compound (2), the base (II) and (1E)-1,3-dichloro-1-propene are added to the aprotic solvent (II) in this order or by a method (b-2) in which the base (II), the compound (2) and (lE)-1,3-dichloro-1-propene are added to the aprotic solvent (II) in this order.

In the step (b), the reaction temperature is preferably from +30 to +180°C, particularly preferably from +70 to +140°C. The reaction time is preferably from 1 to 30 hours, particularly preferably from 1 to 5 hours. Further, the reaction pressure is preferably atmospheric pressure or above, particularly preferably atmospheric pressure.

In the step (b), a metal iodide such as NaI or KI or a metal bromide such as NaBr or KBr may be added to the reaction system so as to further increase reactivity. However, when the distillation step is carried out after the step (a), it is preferred that the reaction in the step (b) is carried out without adding such an iodide or bromide to the reaction system. In the case where the metal iodide or the metal bromide is added thereto, the amount is preferably from 1 to 100 mol%, particularly preferably from 1 to 10 mol% based on (lE)-1,3-dichloro-1-propene.

As the base (II) in the step (b), a base of the formula M²OR² (wherein M² and R² are the same as defined above) is preferably used, and the same base as the base (I) is preferably used. Also when the base of the formula M²OR² is used, it is preferred to react the compound (2) with the base in the presence of the aprotic solvent (II) and then with (1E)-1,3-dichloro-1-propene in accordance with the method (b-1) or (b-2).

In the reaction using the base of the formula M²OR², an alcohol of the formula R²OH will be produced as a by-product. For example, when NaOCH₃ is used as the base, methanol will be produced as a by-product, and when NaOC₂H₅ is used as the base, ethanol will be produced as a by-product. When the alcohol is produced as a by-product, this alcohol is preferably removed from the reaction system before the reaction with (lE)-1,3-dichloro-1-propene. Namely, in the step (b), it is preferred that the compound (2) is reacted with a base (II) made of a metal alkoxide of the formula M²OR² in the presence of the aprotic solvent (II) and then the alcohol of the formula R²OH as a by-product is removed, followed by the reaction with (lE)-1,3-dichloro-1-propene.

A method for removing the alcohol is preferably removal by distillation. Usually, it is preferred to remove the alcohol under heating before adding (lE)-1,3-dichloro-1-propene. It is preferred that a protic solvent such as the alcohol produced as a by-product is removed before adding (1E)-1,3-dichloro-1-propene from the viewpoint of the conversion and reaction time, though the reaction may proceed even if it remains.

In the step (b), from the viewpoint of suppression of the production of an odor substance as mentioned below, it is preferred to control the amount of an unreacted compound (2) contained in the reaction product, and the proportion of the compound (2) to the total amount of the compound (2) and the compound (3) at the time of analysis by means of gas chromatography is preferably less than 5%, particularly preferably less than 0.1%, further more preferably less than 0.01%.

The work-up treatment of the reaction product after the step (b) is preferably carried out by distillation. In the distillation, it is preferred that the aprotic solvent (II) and the unreacted compound of the formula (2) are distilled off. As a work-up treatment step other than distillation, the same method as the treatments 1 to 3 in the step (a) may be mentioned, but it is preferred that no such work-up treatment is carried out to increase the yield of the final product.

The step (c) is dealkoxycarbonylation for obtaining the compound (4) by replacement (dealkoxycarbonylation) of one of -COOR moieties in the compound (3) with a hydrogen atom. Hereinafter, "dealkoxycarbonylation" means a reaction in which one of the two -COOR moieties in the compound (3) is removed and replaced with a hydrogen atom.

The dealkoxycarbonylation may be carried out by a known method. In the present invention, such a reaction is carried out in the same reaction vessel following the step (b). "Such a reaction is carried out in the same reaction vessel" means that by using the product in the preceding step, as it is, the reaction in the subsequent step is carried out in the same reaction vessel. In the production process of the present invention, it is preferred to carry out the reaction in the step (c) after simply distilling the aprotic solvent (II) off from the reaction product in the step (b). According to such a method, no addition of water is carried out for the work-up treatment, whereby a salt produced as a by-product in the step (b), may be used for the step (c).

The reaction in the step (c) is preferably carried out by heating in a polar solvent in the presence of water and an inorganic salt.

The polar solvent may, for example, be an amide solvent such as DMF, DMA, NMP or DMI, a sulfoxide solvent such as DMSO, a sulfone solvent such as sulfolane or an ether solvent such as DME or THF. As the polar solvent, a single polar solvent or a solvent mixture of two or more polar solvents may be used. As the polar solvent, NMP, DMI or sulfolane is preferred since it secures high reactivity and the polar solvent itself does not decompose during the reaction. However, for efficiency of the reaction, it is preferred to select the polar solvent used as the above aprotic solvent (I) and/or (II), and sulfolane is particularly preferred. However, such a polar solvent may contain a non-polar solvent in an amount not to hinder the progress of the reaction.

The amount of the polar solvent is preferably from 2 to 30 times by mass, more preferably from 5 to 15 times by mass, based on the compound (3).

As the solvent to be used in the step (c), it is preferred to use a polar solvent and it is preferred to replace the aprotic solvent (II) used in the step (b) with a polar solvent suitable for the step (c), since it is possible to achieve the progress of the after-mentioned dealkoxycarbonylation at a sufficient reaction temperature and it is possible to increase the solubility of an inorganic salt in water. For example, when a solvent mixture of sulfolane and toluene is used as the aprotic solvent (II), it is preferred to distil toluene off, then add sulfolane and carry out the reaction in the step (c). In such a case, toluene may not necessarily be substituted completely with sulfolane, but may be substituted in such a ratio that the dealkoxycarbonylation proceeds smoothly.

The inorganic salt is preferably an alkali metal halide such as sodium chloride (NaCl), lithium chloride (LiCl), sodium bromide (NaBr) or potassium chloride (KC1), particularly preferably NaCl, NaBr or LiCl. The amount of the inorganic salt is preferably from 0.5 to 50 times by mol, particularly preferably from 0.5 to 10 times by mol.

In the present invention, in order to carry out the steps (b) and (c) continuously, it is preferred to use an alkali metal chloride produced as a by-product in the step (b), as an inorganic salt in the step (c), without newly adding an inorganic salt. Further, in a case where the steps (a), (b) and (c) are continuously carried out in the same reaction vessel, in addition to the alkali metal chloride produced as a by-product in the step (b), an alkali metal halide produced in the step (a) as a by-product may also serve as an inorganic salt in the step (c). Accordingly, when the step (c) is carried out in the presence of an inorganic salt, it is preferred to use an inorganic salt contained in the reaction system in the step (b), as it is, for the reaction in the step (c). In such a case, it is not required to add the inorganic salt in the step (c). The inorganic salt serves to accelerate the reaction rate in the step (c), whereby it is possible to achieve the dealkoxycarbonylation in a shorter period of time and at a lower temperature than those of conventional methods.

The amount of water to be used in the step (c) is preferably from 0.1 to 50 times by mol, particularly preferably from 0.1 to 3 times by mol based on the compound (3).

The reaction temperature for the dealkoxycarbonylation is preferably from +140 to +250°C, particularly preferably from +160 to +200°C. The reaction time is preferably from 3 to 15 hours, particularly preferably from 3 to 5 hours. Further, the reaction pressure is preferably atmospheric pressure or above, particularly preferably atmospheric pressure.

In the present invention, if the compound (2) remains in the reaction system after completion of the step (b), an odor substance is likely to be formed from the compound (2) at the time of the dealkoxycarbonylation in the step (c). Accordingly, it is preferred that the compound (2) is removed from the reaction system after completion of the step (b). It is possible to prevent the production of the odor substance by such removal. On the other hand, the reaction product in the step (b) contains an inorganic salt useful in the step (c). Accordingly, it is preferred to employ work-up treatment which removes the compound (2) but does not remove the inorganic salt. Such work-up treatment is preferably a method by means of distillation. It is possible to carry out the distillation, for example, by reducing the pressure in the reaction system to about 0.4 kPa and then heating the internal temperature to from about 100 to 130°C. Further, when the removal by distillation is carried out after the step (a), it is not required to newly add an inorganic salt since an inorganic salt produced in the steps (a) and (b) is used as a reaction accelerator in the step (c), and also it is possible to shorten the reaction time since the reaction rate of the dealkoxycarbonylation in the step (c) is thereby improved.

It is possible to confirm the amount of the compound (2) by gas chromatography (GC).

The amount of the compound (2) after completion of the step (b) is preferably at most 1%, particularly preferably at most 0.1% based on the reaction product.

In the production process of the present invention, the compound (4) produced in the step (c) is preferably subjected to usual work-up treatment or purification treatment to meet the purpose.

The work-up treatment and/or the purification treatment may, for example, be the following treatments 4 to 6, and treatment 6 is preferred.
(Treatment 4): a method which comprises filtrating the reaction product, quenching the filtrate with water or an aqueous sodium chloride solution and then adding a water-immiscible organic solvent such as dichloromethane, toluene, ethyl acetate or an ether for extraction, concentrating the organic layer, and distilling the organic layer for isolation.
(Treatment 5): a method which comprises cooling the reaction product to in the vicinity of from 50 to 80°C, filtrating the reaction product, and distilling the filtrate under reduced pressure for isolation.
(Treatment 6): a method which comprises cooling the reaction product to in the vicinity of from 50 to 80°C, and then distilling it under reduced pressure for isolation.

Further, in such treatments, by adding an adsorbent such as activated carbon, it is possible to remove impurities such as tarry substances contained in the reaction system.

The production process of the compound (4), which comprises the steps (a) to (c) is preferably a production process which comprises the following (a') to (c').
Step (a'): a step for obtaining the compound (2) by reacting a compound (1) with an alkali metal alkoxide (I) of the formula M¹OR¹ (wherein M¹ is Na or K, and R¹ is a lower alkyl group) in the presence of an aprotic solvent (I) and then with an isopropyl halide to obtain a reaction product containing the compound (2), and then distilling off the aprotic solvent (I) from the reaction product.
Step (b'): a step for obtaining a compound (3) by reacting the compound (2) obtained in the step (a') with the alkali metal alkoxide (I) in the presence of an aprotic solvent (I), and then with (1E)-1,3-dichloro-1-propene to obtain a reaction product containing the compound (3), and then distilling off the aprotic solvent (I) from the reaction product.
Step (c'): a step of carrying out a reaction to replace one of -COOR (wherein R is a lower alkyl group or an aralkyl group) moieties in the compound (3) in the presence of an alkali metal chloride of the formula M¹C1 (wherein M¹ is the same as defined above).

The compound (4) obtained by the above process, namely the (4E)-5-chloro-2-isopropyl-4-pentenoate, is a known compound useful as an intermediate for an agrochemical or a medicine. The compound (4) is especially useful as an intermediate for an insecticide or an antihypertensive agent (WO01/9079).

The compound (4) obtained by the above process is usually a racemate. The compound (4) may be subjected to optical resolution (step (d)), as the case requires. The step (d) is a step for obtaining the following compound (5) and/or (6) by the optical resolution of the compound (4), wherein R is the same as defined above.

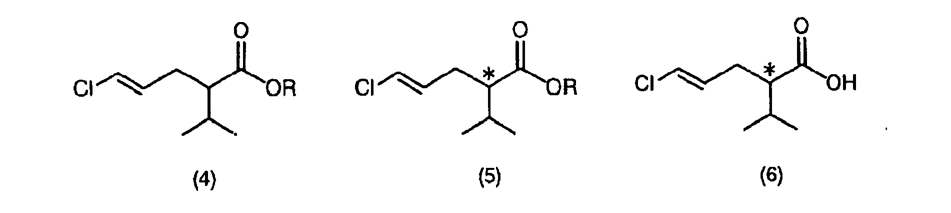

As a method for the optical resolution, a known method may be employed. It is preferred to employ a method comprising treating the compound (4) with a lipase or esterase to selectively hydrolyze one of the optically active isomers since such a method gives the desired compound (5) and/or (6) in a high yield and in a high enantiomeric excess (ee) without requiring special equipment, with easy operations and is suitable for industrial mass production. The method employing a lipase or esterase may be carried out in accordance with the method described in WO04/52828 by the present applicants.

Further, the compound (6) obtainable in the step (d) is converted to the compound (4) by esterification and racemization, whereby it is possible to employ the compound (4) as a starting material in the step (d) again.

In the present invention, it is preferred to obtain the following compound (5a) and/or (6a) by optical resolution employing a lipase or esterase which acts on a racemate of the compound (4). The compound (5a) is a compound more useful as an intermediate for an agrochemical or a medicine, wherein R is the same as defined above.

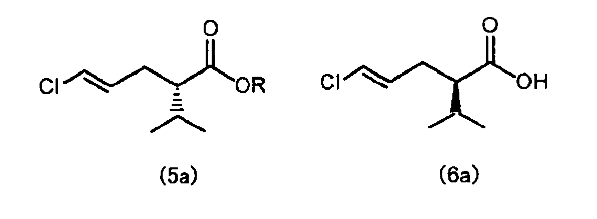

Further, as another method for optical resolution, it is also possible to employ a method which comprises optically resolving one of the optical isomers of the compound (4) to obtain the compound (5b) and/or (6b), and then reacting the compound (5b) with a compound of the formula ROH (wherein R is a lower alkyl group or an aralkyl group) to obtain the compound (5a).

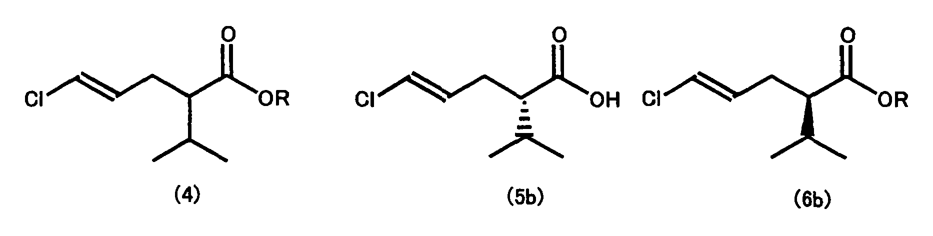

A preferred embodiment of the production process of the present invention is the following process for producing the compound (5aa). Namely, the compound (1aa) is isopropylated to obtain the compound (2aa), then the compound (2aa) is reacted with (lE)-1,3-dichloro-1-propene in the same reaction vessel to obtain the compound (3aa), and one of methoxycarbonyl groups (-CO-OCH₃) in the compound (3aa) is dealkoxycarbonylated in the same reaction vessel to obtain the compound (4aa) in a high yield without conversion to the isomer. The compound (4aa) obtained is subjected to optical resolution using a lipase to obtain the compound (5aa) in a high yield and in a high ee.

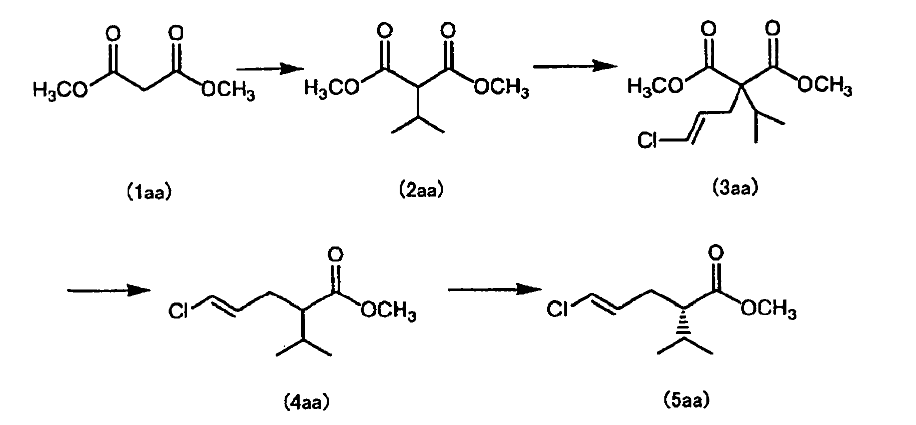

Further, when the work-up treatment after each step in the production process of the present invention, is carried out by distillation of the solvent, it is possible to carry out the work-up treatment without production of waste water as a by-product which was heretofore produced in a large amount. Further, heretofore, it was difficult to recover a polar reaction-solvent such as sulfolane since it was dissolved in waste water, but it is possible to recover a reaction-solvent such as sulfolane or toluene by distillation. Such a method has no problems of waste liquid due to the work-up treatment, and also is capable of recovering and reusing the reaction solvent, whereby such a method affects the environment little and is economically excellent.

### EXAMPLES

Now, the present invention will be described in detail with reference to Examples, but the present invention is by no means restricted to these Examples. Hereinafter, gas chromatography will be referred to as GC, and the amount of an enzyme is given in "Units". 1 Unit is defined as the activity of an enzyme required to convert 1 µmol of ethyl butyrate to 1 µmol of butyric acid per 1 Unit at +25°C and at pH of 8.0. Further, the structures of the compounds obtained were identified by referring to previously available data. The enantiomeric purity and the enantiomeric excess were determined by GC using Lipodex E 50 m x 0.25 mm (Macherey-Nagel) as the column.

### EXAMPLE 1: Example (1) of synthesis of methyl (4E)-5-chloro-2-isopropyl-4-pentenoate

### Step (a):

To a reaction vessel equipped with a stirrer, a thermometer, a condenser and a distilling apparatus, 28% CH₃ONa in methanol (312.8 g), toluene (336 g) and sulfolane (630 g) were added. Then, dimethyl malonate (210 g) in toluene (609 g) was dropwise added thereto. The mixture obtained was gradually heated to distill off the methanol formed as a by-product and the methanol contained in the methanol solution of CH₃ONa. The heating was stopped when the methanol content of the distillate became 0.1% or below, by GC analysis. After the system was cooled to from 95 to 105°C, isopropyl bromide (254.2 g) in toluene (273 g) was added, and then the system was heated with stirring for 15 hours while the internal temperature was kept at from 95 to 105°C. By GC analysis, disappearance of the majority of dimethyl malonate and production of dimethyl 2-isopropyl malonate were confirmed. After addition of toluene (363.3 g), the system was gradually heated to remove the remaining isopropyl bromide together with toluene.

### Step (b):

After completion of the step (a), the system was cooled to 95 to 105°C, and 28% CH₃ONa in methanol (312.8 g) was dropwise added thereto, and then the methanol was distilled off in the same manner as in the step (a).

The heating was stopped when the methanol content of the distillate became 0.01% or below by GC analysis. After the system was cooled to 95 to 105°C, (1E)-1,3-dichloro-1-propene (192.3 g) in toluene was added thereto (here, the amount of toluene was adjusted to be an amount where the mixed ratio of sulfolane and toluene as solvents in the step (b) would be 1/1 (volume ratio), considering the amount of toluene removed in the step (a)). Then, the system was heated with stirring for 2 hours, and the production of dimethyl 2-[(2E)-3-chloro-2-propenyl]-2-isopropyl malonate was confirmed by GC analysis. After the system was cooled to 50°C, the pressure was adjusted at 6.67 kPa, toluene was removed and sulfolane (840 g) was added thereto. Further, after the pressure in the reaction system was adjusted at 0.4 kPa, the system was heated to distill off unreacted dimethyl 2-isopropyl malonate to a level of 0.01% or below, and then the reaction system was returned to atmospheric pressure.

### Step (c):

Sulfolane (420 g) and water (57.2 g) were added to the system after completion of the step (b), and the system was heated to 190°C, followed by reacting for 5 hours. Completion of the reaction was confirmed by GC, and then distillation under reduced pressure was carried out to obtain methyl (4E)-5-chloro-2-isopropyl-4-pentenoate (215.9 g, yield: 71%).

### EXAMPLE 2: Example (2) of synthesis of methyl (4E)-5-chloro-2-isopropyl-4-pentenoate

In the same manner as in Example 1, the step (a) is carried out and followed by the step (b) in the same reaction vessel. Production of dimethyl 2-[(2E)-3-chloro-2-propenyl]-2-isopropyl malonate is confirmed by GC analysis, followed by adding sulfolane (1,260 g) and water (57.2 g). The reaction and the work-up treatment are carried out in the same manner as in the step (c) in Example 1 to obtain methyl (4E)-5-chloro-2-isopropyl-4-pentenoate. The yield is 78% or higher.

### EXAMPLE 3: Example of optical resolution of (4E)-5-chloro-2-isopropyl-4-pentenoate by means of enzyme

614 Units of porcine liver esterase (Roche Diagnostics, Technical Grade) is added to a phosphate buffer (pH 7.0, 5 mmol/L, 230 mL), and the temperature is adjusted to 35 to 40°C. To this solution, racemic methyl (4E)-5-chloro-2-isopropyl-4-pentenoate (20 g) obtained in the same manner as in Example 1 is fed at a rate of 0.065 g/min by means of a tubing pump.

At that time, the reaction system is stirred with a stirring blade to disperse the starting material throughout the reaction system. After the starting material is fed continuously over 5 hours and 10 minutes, the reaction is continued for 21 hours under the same conditions. The starting material is fed to a total concentration of 8 mass%. Further, the pH of the reaction solution is adjusted to 8.0 with 0.5 mol/L aqueous NaOH prior to the feeding of racemic methyl (4E)-5-chloro-2-isopropyl-4-pentenoate (20 g) and kept at 8.0 by means of a pH controller until completion of the reaction. The reaction product is extracted with t-butyl methyl ether, and the organic solvent layer is washed with 5% aqueous sodium carbonate to transfer (R)-(4E)-5-chloro-2-isopropyl-4-pentenoic acid into the aqueous phase. It is found by GC analysis that methyl (S)-(4E)-5-chloro-2-isopropyl-4-pentenoate is obtained in the organic solvent layer in a 90% yield and has an enantiomeric purity of at least 98 %ee.

### COMPARATIVE EXAMPLE: Synthesis of methyl (4E)-5-chloro-2-isopropyl-4-pentenoate

### Step (a):

To a reaction vessel equipped with a stirrer, a thermometer, a condenser and a distilling apparatus, 28% CH₃ONa in methanol (52.1 g), toluene (152 g) and sulfolane (62.3 g) were added. Then, dimethyl malonate (35.0 g) in toluene (46 g) was dropwise added thereto. The mixture obtained was gradually heated to distill off the methanol formed as a by-product, methanol contained in the methanol solution originating from CH₃ONa and toluene. The heating was stopped when the methanol content of the distillate became 0.1% or below, by GC analysis. After the system was cooled to from 95 to 105°C, isopropyl bromide (42.4 g) in toluene (46 g) was added, and then the system was heated with stirring for 17 hours while the internal temperature was kept at from 95 to 105°C. By GC analysis, disappearance of the majority of dimethyl malonate and production of 2-isopropyldimethyl malonate were confirmed. After addition of toluene (61 g), the system was gradually heated to distill off the remaining isopropyl bromide together with toluene.

### Step (b):

After completion of the step (a), the system was cooled to 65 to 95°C, and 28% CH₃ONa in methanol (50.1 g) was dropwise added thereto, and then the methanol and toluene were distilled off in the same manner as in the step (a).

The heating was stopped when the methanol content of the distillate became 0.01% or below by GC analysis. After the system was cooled to 95 to 105°C, (1E)-1,3-dichloro-1-propene (32.1 g) in toluene was added thereto (here, the amount of the toluene was adjusted to be an amount where the mixed ratio of sulfolane and toluene as solvents in the step (b) would be 1/1 (volume ratio), considering the amount removed in the step (a)). Then, the system was heated with stirring for 2 hours, and the production of dimethyl 2-[(2E)-3-chloro-2-propenyl]-2-isopropyl malonate was confirmed by GC analysis. After the system was left to cool to room temperature, the product was added to water (210 g) at 40°C or lower. Liquid separation was carried out to remove waste water (285.6 g). The organic layer was washed with a 7% sodium chloride aqueous solution (117.6 g) to remove waste water (128.2 g). Under reduced pressure, concentration was carried out at 70°C or lower to obtain 130 g of the desired product in the step (b).

### Step (c):

Sulfolane (490 g), water (4.77 g) and sodium chloride (5.42 g) were added to the desired product (130 g) in the step (b), and the system was heated to 210 to 220°C, followed by a reaction for 13 hours. Completion of the reaction was confirmed by GC, and distillation under reduced pressure was carried out to obtain methyl (4E)-5-chloro-2-isopropyl-4-pentenoate (33.3 g, yield: 66%) .

### INDUSTRIAL APPLICABILITY

The present invention provides a process for producing a (4E)-5-chloro-2-isopropyl-4-pentenoate useful as an intermediate for an agrochemical or a medicine in a high yield and efficiently. Further, it provides a process for producing a (S)-(4E)-5-chloro-2-isopropyl-4-pentenoate more useful as an intermediate for an agrochemical or a medicine in a high yield and in a high ee by optical resolution of the (4E)-5-chloro-2-isopropyl-4-pentenoate obtained by the above process. The processes of the present invention are capable of achieving recovery/reuse of the reaction solvent, reduction of waste water and waste solvent, and remarkably high yield, and thus, they are economically excellent. Further, the processes of the present invention are practical for industrial production since they can suppress generation of an odor.

The entire disclosure of Japanese Patent Application No. 2004-301870 filed on October 15, 2004 including specification, claims, drawings and summary is incorporated herein by reference in its entirety.

## Claims

1. A process for producing a compound of the following formula (4), which comprises reacting a compound of the following formula (2) with a base (II) in the presence of an aprotic solvent (II), and then with (lE)-1,3-dichloro-1-propene in the same reaction vessel to obtain a compound of the following formula (3), and then carrying out a reaction to replace one of -COOR moieties in the compound of the following formula (3) with a hydrogen atom in the same reaction vessel: 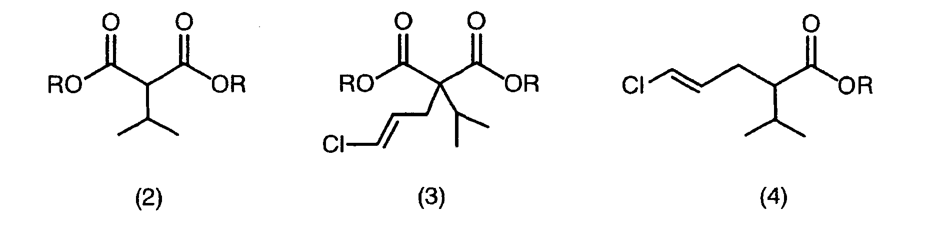 wherein R is a lower alkyl group or an aralkyl group.

2. The process according to Claim 1, wherein the reaction for conversion from the compound of the formula (3) to the compound of the formula (4), is carried out after the aprotic solvent (II) is distilled off from the reaction product obtained by the reaction with (lE)-1,3-dichloro-1-propene.

3. The process according to Claim 2, wherein the reaction for conversion from the compound of the formula (3) to the compound of the formula (4), is carried out in the presence of a salt produced by the preceeding reaction.

4. The process according to any one of Claim 1, 2 or 3, wherein the compound of the formula (2) is a compound obtained by reacting a compound of the following formula (1) with a base (I) in the presence of an aprotic solvent (I), and then with an isopropyl halide: 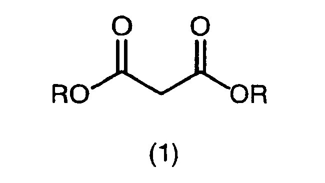 wherein R is the same as defined above.

5. The process according to Claim 4, wherein the reaction of the compound of the formula (2) with the base (II), is carried out after the aprotic solvent (I) is distilled off from the reaction product obtained by the reaction with the isopropyl halide.

6. The process according to Claim 4 or 5, wherein after the reaction to obtain the compound of the formula (2), the reaction to obtain the compound of the formula (3) is carried out in the same reaction vessel.

7. The process according to Claim 4, 5 or 6, wherein the base (I) and the base (II) are the same base.

8. The process according to Claim 4, 5, 6 or 7, wherein the aprotic solvent (I) and the aprotic solvent (II) are the same solvent.

9. A process for producing a compound of the following formula (4), which comprises the following steps (a'), (b') and (c'):
step (a'): a step of reacting a compound of the following formula (1) with an alkali metal alkoxide (I) of the formula M¹OR¹ (wherein M¹ is Na or K, and R¹ is a lower alkyl group) in the presence of an aprotic solvent (I) and then with an isopropyl halide to obtain a reaction product containing a compound of the following formula (2), and then distilling off the aprotic solvent (I) from the reaction product,
step (b'): a step of reacting the compound of the formula (2) obtained in the step (a') with the alkali metal alkoxide (I) in the presence of an aprotic solvent (I), and then with (1E)-1,3-dichloro-1-propene to obtain a reaction product containing a compound of the following formula (3), and then distilling off the aprotic solvent (I) from the reaction product, and
step (c'): a step of carrying out a reaction to replace one of -COOR moieties in the compound of the formula (3) obtained in the step (b') with a hydrogen atom, in the presence of an alkali metal chloride of the formula M¹C1 (wherein M¹ is the same as defined above) : 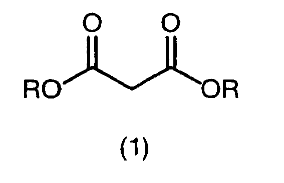 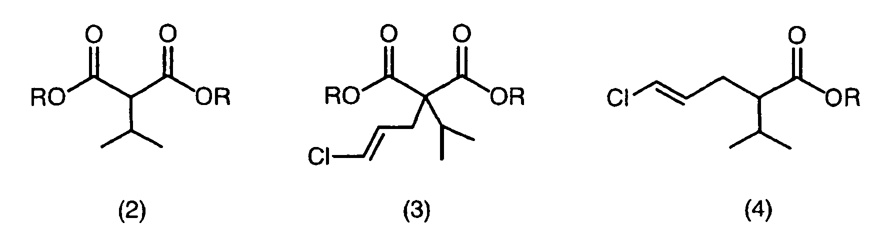
wherein R is a lower alkyl group or an aralkyl group.

10. The process according to Claim 9, wherein the step (c') is a step which comprises heating the compound of the formula (3) obtained in the step (b'), in the presence of an alkali metal chloride of the formula M¹C1 (wherein M¹ is the same as defined above) formed in the step (b') without adding the alkali metal chloride and in the presence of water.

11. A process for producing a compound of the following formula (5a) and/or a compound of the following formula (6a), which comprises optically resolving the compound of the following formula (4) obtained by the process as defined in any one of Claims 1 to 10: 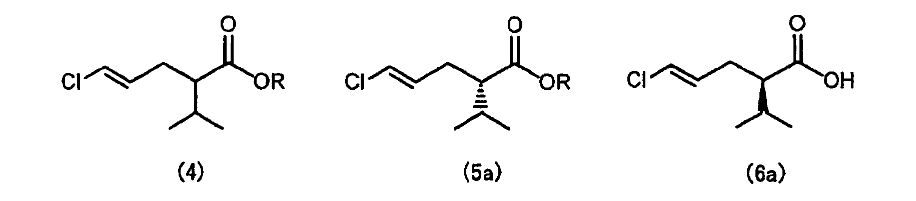 wherein R is the same as defined above.

12. A process for producing a compound of the following formula (5b) and/or a compound of the following formula (6b), which comprises separating, by optical resolution, one of optical isomers of the compound of the following formula (4) obtained by the process as defined in any one of Claims 1 to 10: 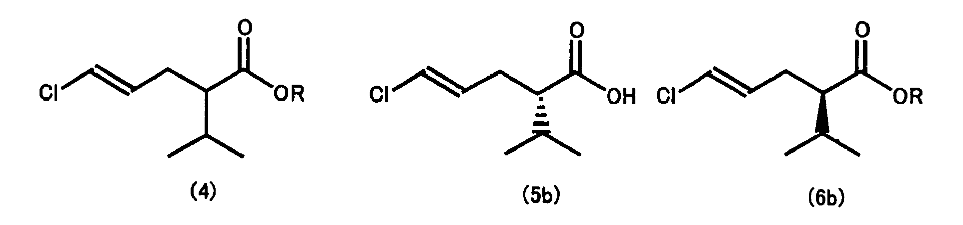 wherein R is the same as defined above.

13. A process for producing a compound of the following formula (5a), which comprises reacting a compound of the formula ROH with the compound of the formula (5b) obtained by the process as defined in Claim 12: 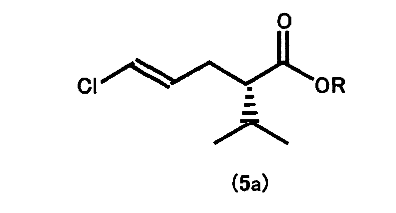 wherein R is a lower alkyl group or an aralkyl group.

14. The process according to Claim 11, 12 or 13, wherein the optical resolution is carried out by letting an enzyme act on the compound of the following formula (4):
